# EUROPEAN PATENT APPLICATION

(11) **EP 4 585 197 A1**
(43) Date of publication of application: **16.07.2025**
(21) Application number: 24151314.2
(22) Date of filing: 11.01.2024
(51) Int. Cl.: A61F 13/49, A61F 13/532, A61F 13/534, A61F 13/535, A61F 13/539, A61F 13/56

(54) **ABSORBENT ARTICLE COMPRISING A CHANNEL WITH VARYING BOND STRENGTH**

(71) Applicant: The Procter & Gamble Company, Cincinnati, OH 45202 (US)
(72) Inventor: Garcia, Julien René, 65824 Schwalbach am Taunus (DE); Peri, Andrea, 65824 Schwalbach am Taunus (DE); Shakaroun, Hossein, 65824 Schwalbach am Tauns (DE)
(74) Representative: P&G Patent Germany

(57) **Abstract**

A personal hygiene absorbent article (20) comprising at least one elongated, absorbent material-free channel (26) having a front end (62) and a back end (66) and a middle point (64). The channel comprises a channel bond (27) between the core wrap top layer (45) and the core wrap bottom layer (46). The channel bond strength varies along the length of the channel so that the bond strength in an intermediate section (102) is weaker than the bond strength in a front section (100)and a crotch section (104). The bond strength is measured in minutes for each section according to the Static Peel Force Time Test disclosed herein.

## Description

### FIELD OF THE INVENTION

The invention relates to personal hygiene absorbent articles such as diapers that comprise one or more channels in the absorbent core.

### BACKGROUND OF THE INVENTION

Modern personal hygiene absorbent articles such as baby diapers and adult incontinence diapers are manufactured by converting multiple layers and components at high speed. Such absorbent articles comprise a fluid-permeable topsheet on the wearer-facing side of the article, a fluid-impermeable backsheet on the garment-facing side of the article, and an absorbent core disposed in-between these two layers. The absorbent core typically comprises an absorbent material disposed in a core wrap. The absorbent material is typically a mixture of cellulose fibers and superabsorbent polymer particles ("SAP"). Over the years, the SAP content has increased relative to the cellulose fibers, thus providing thinner absorbent cores. Absorbent cores without cellulose fibers, so called airfelt-free cores, have also been recently proposed. Other typical components of diapers include an acquisition layer between the topsheet and the absorbent core, as well as inner and outer leg cuffs. Some absorbent articles comprise a dual layer acquisition-distribution system, especially with airfelt-free absorbent cores.

Many personal hygiene articles, in particular baby diapers, now comprise one or more elongated absorbent material-free channels in the absorbent core. These channels can help quickly distributing urine along their length to better use the absorbent capacity of the absorbent core. The top layer of the core wrap may be bonded to the bottom layer of the core wrap in the channel to make the channels more durable against the delamination pressure of the swollen neighboring superabsorbent material. Exemplary disclosures of core with permanent or semi-permanent channels can be found in WO2012/170778 (Rosati et al.) and US2012/0312491 (Jackels).

Channels in the absorbent core can also improve the flexibility of the diaper, reduce wet sagging and improve liquid distribution. However, at certain load, the channels can constrain the core, make fluid absorption slower especially in the crotch where the SAP is more concentrated and most utilized. The absorbent core's effective capacity can be lowered and also the kinetic of absorption may be reduced.

Various improvement to channel designs have been proposed in the patent literature. For example WO2019/83711A1 (Bianchi) discloses an absorbent article comprising at least two discrete channels within the absorbent material. A first type of channel is provided where the top side and bottom side of the core wrap are bonded together, and a second type of channel where the top and bottom sides are not bonded or significantly less bonded than in the first type. The channel bonding can be achieved through adhesive bonding, thermo bonding, mechanical bonding, ultrasonic bonding, or a combination of these methods. The first type of channels has a higher static peel force time compared to the second type.

Discrete channels can however compromise the benefit of liquid spreading and wet fit.

### SUMMARY OF THE INVENTION

The invention is for a personal hygiene absorbent article comprising a front waist region, a crotch region, a back waist region, a longitudinal centerline extending in a longitudinal direction (y) and a length L measured along the longitudinal centerline, wherein the front waist region, crotch region and back waist region have the same length of one third of L as measured along the longitudinal centerline. The article comprises a topsheet, a backsheet and an absorbent core between the topsheet and the backsheet. The absorbent core comprises a core wrap top layer, a core wrap bottom layer, and an absorbent material layer between the core wrap top layer and bottom layer, wherein the absorbent layer has a length L' measured in the longitudinal direction along the longitudinal centerline.

The absorbent core comprises at least one elongated, absorbent material-free channel having a front end and a back end and a middle point, wherein the middle point is disposed longitudinally equidistantly between the front end and the back end of the channel. The channel is at least partially present in the crotch region of the article, and the channel has a length L" measuring the longitudinal direction of at least half of L'.

The channel comprises a front section encompassing or adjacent the channel's front end, a crotch section encompassing or adjacent the channel's middle point and an intermediate section between the front section and the crotch section. The bond strength varies along the length of the channel so that the front section and crotch section each have a channel bond strength which is higher than the bond strength in the intermediate section. The channel bond in the front section may further have a higher strength than in the crotch section. The bond strength is measured in minutes according to the Static Peel Force Time Test disclosed hereinbelow, wherein the bond strength is measured on a 2.52 cm cutout in each section as disclosed therein.

The absorbent core of the invention comprises at least one channel having sections of varying bond strength as disclosed herein. If the core comprises a pair of elongated channels symmetrically disposed relative to the longitudinal axis, each of the channel in the pair of channels may advantageously be a channel according to the invention. The absorbent core of the invention may also comprise one or more additional channels having a different bond strength pattern than the channel claimed.

By providing a weaker intermediate channel bond strength between the front section and the crotch section of the channel, the swelling pressure caused by the swelling absorbent material can be released at this intermediate section by preferentially delaminating the channel bond in the intermediate section. The channel bond can thus remain longer in the front section and the central section. The present invention can provide better fluid handling, and in particular help reducing leakage and/or reduce absorbent material usage.

### BRIEF DESCRIPTION OF THE DRAWINGS

Fig. 1 is a top view of the wearer-facing side of an exemplary article of the invention in the form of a taped diaper which has been pulled flat, with some layers partially removed;
Fig. 2 shows a cross-section view of the taped diaper of Fig. 1;
Fig. 3 shows an exploded view of the taped diaper of Fig. 1;
Fig. 4 shows a top view of the exemplary absorbent core of the previous Figures shown in isolation, and the top side of the core wrap partially removed;
Fig. 5 shows a schematic view of the absorbent core in exploded view;
Fig. 6 shows the irregular adhesive pattern used to provide the different bonding strength along the channel;
Fig. 6a,b,c show closeup view of the front, medium and intermediate sections of the channel with the adhesive pattern of Fig. 6;
Fig. 7a, b shows a straight channel having zones of different bonding strength according to the invention;
Figs. 8-12 illustrate how to conduct the Static Peel Force Time test further described herein.

### DETAILED DESCRIPTION OF THE INVENTION

### Absorbent article

As used herein, "absorbent articles" refers to personal hygiene devices that are designed to be placed in contact to the body of a wearer to absorb and contain body exudates. The absorbent articles of the invention include baby care articles, feminine care articles or adult incontinence articles.

Baby care articles are products intended for babies, toddlers and/or children, including taped diapers, pant diapers, absorbent inserts, infant and/or toddler care wipes, and infant and/or toddler bibs.

Feminine care articles are products relating to catamenial pads, incontinence pads, interlabial pads, panty liners, pessaries, sanitary napkins, tampons and tampon applicators, and/or wipes.

Adult incontinence articles are products intended for adults, relating to disposable absorbent articles including taped diapers, pant diapers, absorbent inserts, incontinence pads, panty liners, and vaginal inserts.

The absorbent articles of the invention are typically disposable and are preferably recyclable.

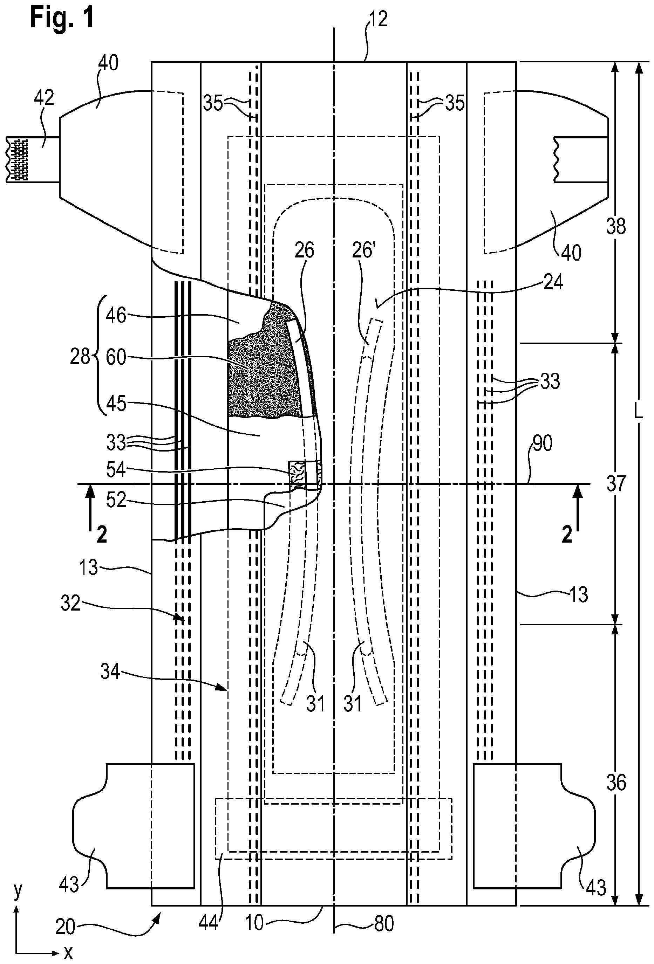

As used herein, "diapers" refers to absorbent articles designed to be worn by babies, infants or incontinent adults about the lower torso, so as to encircle the waist and legs of the wearer, and that are specifically adapted to receive and contain urinary and fecal waste. Diapers are typically proposed as taped diapers or pant diapers. Taped diapers have a fastening system (as illustrated in Fig. 1 for example), where the waist opening and leg openings are formed when the diaper is applied onto the wearer by releasably attaching the longitudinal edges of the front waist region and back waist region to each other. In pant diapers, on the other hand the longitudinal edges of the waist regions are attached to each other to form a pre-formed waist opening and leg openings. A pant diaper is placed in position on the wearer by inserting the wearer's legs into the leg openings and sliding the pant diaper into position about the wearer's lower torso. A pant may be pre-formed by any suitable techniques including, but not limited to, joining together portions of the absorbent article using re-fastenable and/or non-refastenable bonds (e.g., seam, weld, adhesive, cohesive bond, fastener, etc.). A pant may be pre-formed anywhere along the circumference of the article (e.g., side fastened, front waist fastened).

An exemplary, non-limiting absorbent article according to the invention is discussed herein in relation to the Figures 1-2. Figure 1 is a plan view of such an exemplary diaper 20, in a flat-out state, with portions of the diaper being cut-away showing the different layers of the diaper. This diaper 20 is shown for illustration purpose only, as the present invention may be comprised in a wide variety of diapers or other absorbent articles, such as pant diapers having pre-formed side seams. The side seams of pant articles can be opened by cutting or otherwise, if it is desired to place the pant in a flattened-out configuration similar to Fig. 1.

As illustrated in Fig. 1, the absorbent article 20 comprises a front edge 10, a back edge 12, and two longitudinally-extending side (lateral) edges 13, 14. The front edge 10 is the edge of the article which is intended to be placed towards the front of the user when worn, and the back edge 12 is the opposite edge. The absorbent article may be notionally divided by a longitudinal axis 80 extending along a longitudinal direction y from the middle of the front edge 10 to the middle of the back edge 12 of the article. The longitudinal axis thus notionally divides the article in two substantially symmetrical left and right halves relative to this axis, when viewing the article from the wearer-facing side in a flat-out configuration, as exemplarily shown in Fig. 1.

The article has a length L as measured from its front end to its back end along the longitudinal axis. The transversal centerline 90 is the imaginary line perpendicular to the longitudinal centerline 80 in the plane of the flattened-out diaper and notionally dividing the article into a front half and back half having an equal length of L/2.

If some parts of the article are under tension due to elasticized components, the article may be typically flattened using clamps along the periphery of the article and/or a sticky surface, so that the article can be pulled taut to be substantially flat. Closed articles such as pant-like baby diapers, training pants for small children, or adult incontinent pants may be cut open along the side seams to apply them on a flat surface, as is known in the art. Unless otherwise indicated, dimensions and areas disclosed herein apply to the article in this flat-out configuration.

The absorbent article, whether a taped diaper or a pant diaper, can be notionally divided in a front waist region 36, a back waist region 38 opposed to the front waist region 36, and a crotch region 37 located between the front waist region 36 and the back waist region 38. The crotch region, the front waist region and the back waist region are hereby defined as delimiting each one third of the length of the absorbent article along the longitudinal centerline 80.

As illustrated in Figures 1-2, the absorbent article comprises a topsheet 24 on its wearer-facing side, a backsheet 25 on its garment-facing side, and an absorbent core 28 between the topsheet 24 and the backsheet 25. The absorbent core 28 comprises at least one absorbent layer 60 comprising superabsorbent polymer particles ("SAP") and a core wrap. The absorbent layer 60 is disposed between a top core wrap layer 45 and a bottom core wrap layer 46.

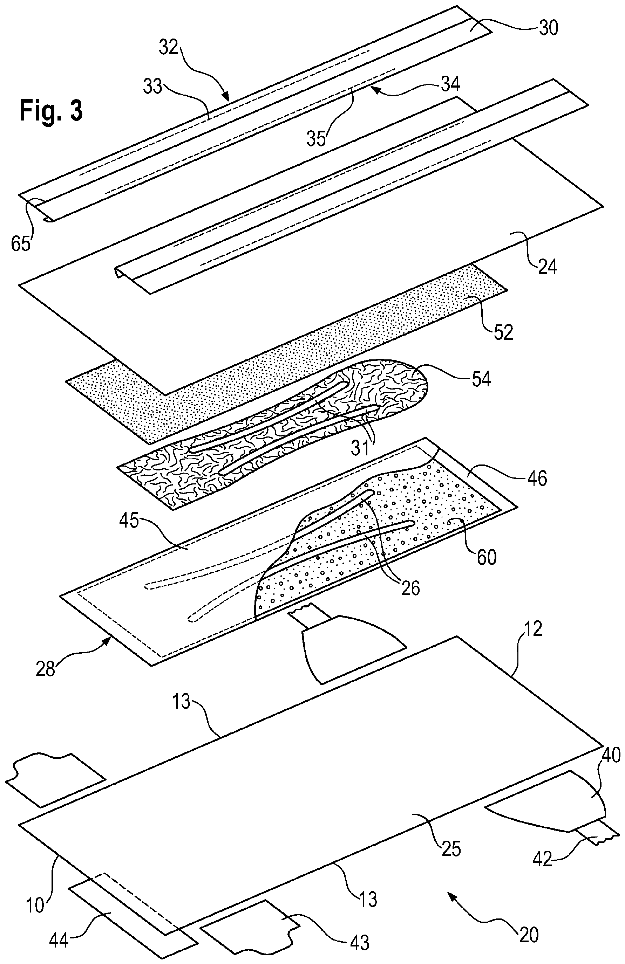

The absorbent layer 60 typically has a pre-determined outline (periphery) as considered in the plane formed by the article when flattened out. This outline may be substantially rectangular as shown on Fig. 3, but the absorbent layer may also have another shape such as a sand-hour or dog-bone shape. The absorbent core 28 further comprises at least one elongated, absorbent material-free channel 26, which is an area substantially free of absorbent material within the absorbent layer 60. The channel is typically elongated in the longitudinal direction, so that it extends more, preferably at least 4 time more in the longitudinal direction y than in the transversal direction x. The channel(s) 26 facilitates the distribution of urine along the length of the absorbent article.

The absorbent articles of the invention may further comprise an acquisition-distribution system between the topsheet and the absorbent core. The acquisition-distribution system may be comprised of a single acquisition layer, or alternatively as represented it may comprise an upper acquisition layer 52 directly underneath the topsheet, and a distribution layer 54 between the acquisition layer and the absorbent core.

An example of acquisition layer 52, which may be used alone or with a distribution layer, may be a surfactant treated, latex bonded, nonwoven acquisition layer. The distribution layer 54 may be a layer of cross-linked cellulose fibers, especially when the absorbent core 28 is an airfelt-free absorbent core. Another example of acquisition layer 52 is a spunlace layer, which may be used alone or in combination with a distribution layer 54.

Nonwovens are sheet or web structures bonded together by entangling fibers or filaments mechanically, thermally, or chemically. They are flat, porous sheets that are made directly from separate fibers. They are not made by weaving or knitting and do not require converting the fibers to yarn. Common process to make nonwovens are meltblowing, spunbonding, solvent spinning, electrospinning, carding and airlaying. The basis weight of nonwoven webs is usually expressed in grams per square meter (g/m² or gsm).

Suitable spunlace nonwovens comprise absorbent fibers, stiffening fibers and resilient fibers, as for example disclosed in WO2020/205485 (Peri et al.). The spunlace nonwoven may typically comprise from about 20 percent to about 75 percent of absorbent fibers, from about 1 percent to about 50 percent of stiffening fibers, and from about 10 percent to about 50 percent of resilient fibers. However other materials having fluid acquisition and distribution properties and integrity may be used.

Further, the absorbent article may comprise other optional but conventional elements, which are not represented for simplicity, such as a back waist elastic feature, a front waist elastic feature, a lotion applied onto the body-facing surface of the topsheet, or a urine indicator disposed on the inner side of the backsheet that changes color when contacted with urine.

The topsheet 24, the backsheet 25, and the absorbent core 28 may be assembled in a variety of well-known configurations, such as by gluing, heat embossing, ultrasonic bonding or combinations thereof. Exemplary diaper configurations are described generally in US3,860,003; US5,221,274; US5,554,145; US5,569,234; US5,580,411; and US6,004,306.

### Topsheet

The topsheet 24 forms the wearer-facing surface of the article that is in intimate contact with the skin of the wearer, in particular the perineal area. At least a portion of and typically all the topsheet is liquid permeable, permitting liquid bodily exudates to readily penetrate through its thickness. The topsheet may consist or comprise a carded calendered nonwoven according to the invention, benefitting from the excellent softness of the nonwoven of the invention, especially when the staple fibers comprise a combination of natural and synthetic fibers.

The topsheet may have one or more layers. The topsheet may be apertured or non-apertured, and may have any suitable three-dimensional features, and/or may have a plurality of embossments (e.g., a bond pattern). Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet.

### Backsheet

The backsheet 25 is generally the portion of the absorbent article 20 that constitutes all or a part of the garment-facing surface of the absorbent article. The backsheet 25 may be joined at least partially to the topsheet 24, the absorbent core 28, or other layers of the article by any attachment methods known to those of skill in the art. The backsheet prevents, or at least inhibits, the bodily exudates absorbed and contained in the absorbent core from soiling articles such as bedsheets, undergarments, and/or clothing. The backsheet is typically liquid impermeable, or at least substantially liquid impermeable.

The backsheet typically comprises a thin impermeable plastic film, usually a thermoplastic film having a thickness of about 0.01 mm to about 0.05 mm. The backsheet material may be breathable, which permit vapors to escape from the absorbent article, while still preventing, or at least inhibiting, bodily exudates from passing through the backsheet. A breathable backsheet may have a Water Vapor Transmission Rate (WVTR) of from 1,000 to 15,000 g/m²/24h, or from 1,000 to 10,000 g/m²/24h, or from 1,500 to 10,000 g/m²/24h as measured using a PERMATRAN-W Model 101K (available from Mocon, Inc., Minneapolis, MN) or equivalent, according to Nonwovens Standard Procedure NWSP 70.4.R0(15) with the following specifications: experiments were carried out in a lab controlled at 23 °C ± 2 C° and 50 %RH ± 2 %RH and the instrument cells heated to 37.8°C (100°F).

The backsheet 25 may also comprise a backsheet outer cover nonwoven (not represented separately in the Figures). The backsheet outer cover nonwoven is typically a thin nonwoven material that is joined to the outer surface of the backsheet film. The outer cover nonwoven may thus form the garment-facing surface of the backsheet. The backsheet may thus comprise a carded calendered nonwoven according to the invention. Especially the nonwoven of the invention may form the backsheet outer cover, profiting from the improved softness properties of the nonwovens of the invention. The backsheet outer cover nonwoven may also comprise a bond pattern, apertures, and/or three-dimensional features, and may improve the feel of the backsheet.

### Barrier cuffs 32, 34

The absorbent article 20 may also comprise inner barrier leg cuffs 34 and outer leg cuffs 32, as is known in the art. The inner barrier cuffs 34 can extend upwards from the surface of the article to provide retention of the waste, while the outer cuffs are typically formed in the plane of the chassis of the article as defined by topsheet and backsheet. These cuffs are preferably elasticized, as is known in the art, for example using elastic threads 33, 35 as represented in the Figures 1-2. While not illustrated, the absorbent article may also be in the form of a pant diaper comprising an inner and outer belt nonwovens as is known in the art, in which case the nonwoven of the invention may also be comprised in any of the inner and outer belt nonwovens.

Moreover, the absorbent article may comprise a fastening system, such as an adhesive fastening system or a hook and loop fastening member, which can comprise tape tabs 42 disposed on back ears 40, such as adhesive tape tabs or tape tabs comprising hook elements, cooperating with a landing zone 44 (e.g. a nonwoven web providing loops in a hook and loop fastening system). While taped diapers typically comprise back ears 40, and front ears 43, these are typically not present in pant-type absorbent articles having pre-formed side seams.

The front and/or back ears may be separate components attached to the absorbent article or may instead be continuous with portions of the topsheet and/or backsheet such that these portions form all or a part of the front and/or back ears 40, 43. Also combinations of the aforementioned are possible, such that the front ears 43 and/or back ears 40 are formed by portions of the topsheet and/or backsheet while additional materials are attached to form the overall front and/or back ears 40, 43. The front and/or back ears may be elastic or non-elastic. Also, the front ears 40 may be applied as separate components attached to the absorbent article while the back ears (or parts thereof) may be continuous with portions of the backsheet and/or topsheet - or vice versa.

### Liquid management layers 52, 54

The absorbent article may advantageously comprise at least one liquid management layer 52, also referred herein as acquisition layer and/or distribution layer, between the topsheet 24 and the absorbent core 28 as illustrated in Figs. 1-2. Liquid management layers quickly acquire and/or distribute the fluid away from the topsheet and into the core. These liquid management layers are sometimes called "wicking layer", "surge layer", "acquisition layer", "distribution layer" or "acquisition-distribution layer". Typically, liquid management layers do not comprise SAP, as this may slow the acquisition and distribution of the fluid. The prior art discloses many type of liquid management layer, see for example WO2000/59430 (Daley), WO95/10996 (Richards), US5,700,254 (McDowall), WO02/067809 (Graef). Liquid management layers are typically placed symmetrically relative to the longitudinal axis of the article.

The liquid management layer 52 may be made of a nonwoven web having hydrophilic properties, often referred to as an acquisition layer. The nonwoven web may be for example provided as a continuous roll of material that is cut according to the desired length and pattern as it is unwound in a converting line. Such an acquisition layer is typically placed directly under the topsheet. Suitable nonwovens are for example through-air bonded ("TAB") carded nonwovens, resin-bonded ("RB") carded nonwovens, spunbond or spunlace (hydroentangled) nonwovens. TAB carded nonwovens may for example be made from soft PE/PP bicomponent staple fibers. The air through bonding process locks in loft and compressibility. Resin-bonded carded nonwovens may be made from multi-denier polyester staple fibers (for example: 50/50 or 40/60 mix of 6 denier and 9 denier fibers). Its resilient and open structures are designed to provide excellent fluid acquisition properties. Such acquisition layers are available directly from suppliers, e.g. Fitesa of Simpsonville, South Carolina, USA or TWE Group GmbH, of Emsdetten, Germany. The nonwoven layer may be stabilized by a latex binder for example a styrene-butadiene latex binder (SB latex). Processes for obtaining such latexes are known, for example from EP149,880 (Kwok), US2002/028858 and US2003/0105190 (Diehl). The binder may typically be present in an acquisition layer in excess of about 12%, about 14% or about 16% by weight of the layer. A SB latex is for example commercially available under the trade name GENFLO^{™} 3160 (OMNOVA Solutions Inc.; Akron, Ohio). Latex bonded acquisition layers are for example further disclosed in US2005/033252A1, US2005/033253A1 or US2005/043694A1 (Schneider). The basis weight of typical acquisition layers ranges from 10 gsm to 200 gsm, in particular 20 gsm to 140 gsm, or 40 gsm to 120 gsm, for example 80 gsm.

Since the function of a liquid management layer is to help transfer the insulting fluid to the absorbent material, it is generally not desirable for the liquid management to extend beyond the edges of the absorbent material area of the core. The liquid management layer may be typically rectangular, and its width may be shorter in the transversal direction than the minimum width of the absorbent material layer of the absorbent core.

When the absorbent material comprises, cellulose fibers mixed with the SAP, a single liquid management layer 52 in the form a hydrophilic nonwoven acquisition layer may be sufficient. Cellulose fibers can typically help acquiring and distributing the fluid within the core. Where the absorbent material of the core is substantially free of cellulose fibers however, it may be advantageous to have two liquid management layers, in the form of the combination of an acquisition layer 52 and a distribution layer 54, as described below and illustrated in any of Figs. 1-3.

The absorbent articles of the invention may comprise a first and a second liquid management layers. These layers may form an integrated unitary layer or remain discrete layers which are attached to each other for example by adhesive bonding. The article may comprise an acquisition layer 52 directly under the topsheet and a distribution layer 54 between the acquisition layer and the absorbent core, as illustrated in any of Figs. 1-3. The distribution layer may comprise cross-linked cellulosic fibers and the acquisition layer comprised of a carded, resin-bonded hydrophilic nonwoven. The distribution layer may be profiled having a narrower crotch or have a generally rectangular. Distribution layer with a rounded end towards the back of the diaper ("bullet" shaped) have been suggested. Such dual layer liquid management layers are for example disclosed in further details in WO2014/093323 (Bianchi et al.). In the example shown in Fig. 1-3, the distribution layer 54 is bullet shaped with a fitted crotch portion have a narrower width relative to the front and back portion of the distribution layer.

Such a fibrous distribution layer 54 may for example be made on-line by depositing the fibers, for example cross-lined cellulosic fibers, on a forming surface having ridges corresponding to the areas where no fibrous material is desired. Deposition chambers are known wherein a carrier sheet is provided on a forming surface having a series of holes connected to a vacuum, so that the vacuum pulls the fibers in the desired emplacements to form a desired deposited layer. The forming surface of these deposition chambers can be modified to provide a layer of fibrous material having a central portion and side portions separated by folding guides. The fibrous layer is typically formed or transferred on a carrier sheet that should thus have at least the same dimension as a fibrous liquid management layer. The carrier sheet may be the topsheet, another liquid management layer such as a nonwoven acquisition layer 52, or any other layer of the article, for example the core wrap.

The function of a distribution layer is to spread the insulting fluid liquid over a larger surface within the article so that the absorbent capacity of the core can be more efficiently used. Typically, distribution layers can be made of a material comprising synthetic or cellulosic fibers and having a relatively low density. The distribution layer material may be a nonwoven or a fibrous layer comprising unbound or loosely bound hydrophilic fibers, in particular a layer of cross-linked cellulosic fibers. The density of the distribution layer may vary depending on the compression of the article but may typically range from 0.03 g/cm³ to 0.25 g/cm³, in particular from 0.05 g/cm³ to 0.15 g/cm³ measured at 0.30 psi (2.07kPa). The distribution layer may also be a material having a water retention value of from 25 to 60, preferably from 30 to 45, measured as indicated in the procedure disclosed in US5,137,537.

Such a distribution layer 54 may for example comprise at least 50% by weight, optionally consisting of 100%, of cross-linked cellulosic fibers. The cross-linked cellulosic fibers may be crimped, twisted, or curled, or a combination thereof including crimped, twisted, and curled. This type of material has been used in the past in disposable diapers as part of an acquisition system, for example US 2008/0312622 A1 (Hundorf), however not in the manner of the invention. The cross-linked cellulosic fibers provide higher resilience and therefore higher resistance against the compression in the product packaging or in use conditions, e.g. under baby weight. This provides the layer with a higher void volume, permeability and liquid absorption, and hence reduced leakage and improved dryness. The liquid management layer 54 may also be typically profiled so that more material is present at the front and middle part of the article relative to the back of the article. The distribution layer may typically have an average basis weight of from 30 to 400 g/m², in particular from 100 to 300 g/m², with the basis weight varying along the length of the article so that more material is present at the front and middle of the layer than at the back. The liquid management layer may thus be profiled and/or shaped rounded towards the back of the article, as exemplarily disclosed in WO2014/093323 (Bianchi).

The distribution layer 54 may be provided with folding guides 31 as illustrated in Figs. 1-3. The folding guides 31 may be at least partially vertically superposed with the channels 26, 26' of the absorbent core, when these are present, as illustrated in Figs. 1-3. Thus, the liquid management layer 54 can easily fold similarly to the core when adapting to a three-dimensional basin shape. "Vertically superposed" means that the position and shape of the folding guides of the liquid management layer vertically correspond to the underlying channel-forming areas of the absorbent core, so that the liquid management layer can readily assume a basin-like shape formed by the underlying absorbent core when the article is put on and worn by the wearer.

The folding guide(s) 31 of the liquid management layer may be superposed with the core channel(s) 26 of the absorbent core over the whole length of folding guides, but a lower percentage of overlap is also possible. For example, the liquid management layer's folding guides may overlap over at least 50%, 60%, 70% or more of the overall length of the absorbent core's channel-forming area(s). In the remaining areas where there is no overlap, the liquid management layer's folding guides may for example be off-set relative to the absorbent core's channel-forming area(s) or may be shorter and thus not extend to the same length as the absorbent core channel(s) 26.

If folding guide(s) 31 are present in such a distribution layer, they may be formed by areas substantially free of the liquid management material, in this case substantially free of unbound or loosely bound hydrophilic fibers such as cross-linked cellulosic fibers as illustrated in Figs. 1-3.

The invention is however not restricted to this example having two liquid management layers. Many disposable absorbent articles have in particular for cost reason only one liquid management layer 52. As indicated previously, there may also be no liquid management layer between the absorbent core and the topsheet, and/or one such layer may be present under the absorbent core, between the absorbent core and the backsheet.

### Absorbent core 28

The absorbent articles of the invention comprise an absorbent core between the topsheet and the backsheet. The absorbent core 28 comprises an absorbent layer 60 and a core wrap comprising a core wrap top layer 45 and a core wrap bottom layer 46. The absorbent layer typically comprises superabsorbent polymer particles (SAP) optionally mixed with cellulose fibers. SAP are water-insoluble, water-swellable polymers capable of absorbing large quantities of fluids, as is known in the art. The term "superabsorbent polymer" refers herein to absorbent materials, typically cross-linked polymeric materials, which can absorb at least 10 times their weight of an aqueous 0.9% saline solution as measured using the Centrifuge Retention Capacity (CRC) test as indicated in EDANA method NWSP 241.0.R2 (19). The SAP may in particular have a CRC value of more than 20 g/g, or more than 24 g/g, or of from 20 g/g to 50 g/g, or from 20 g/g to 40 g/g, or 24 g/g to 35 g/g.

The absorbent layer 60 is typically sandwiched, and preferably at least partially immobilized, between the core wrap top layer 45 and the core wrap bottom layer 46, so that the absorbent core can be easily integrated with the rest of the chassis of the absorbent article in a converting line.

Superabsorbent polymer particles are often mixed with cellulose fibers (airfelt cores). The absorbent core may alternatively comprise at least one layer of SAP, wherein the SAP particles are not mixed with cellulose fibers. The resulting layer of absorbent material may thus have a reduced thickness in the dry state, compared to conventional airfelt-based absorbent cores. The reduced thickness helps to improve the fit and comfort of the absorbent article for the wearer. The absorbent material may be free of cellulose fibers (however the absorbent core may still comprise some cellulose fibers in a nonwoven or tissue layer, such as a spunlace layer, for example as a core wrap layer).

Various absorbent core designs comprising a layer of SAP free of cellulose fibers have been proposed in the past, see for example in US5,599,335 (Goldman), EP1,447,066 (Busam), WO95/11652 (Tanzer), US2008/0312622A1 (Hundorf), WO2012/052172 (Van Malderen). In particular, the SAP printing technology as disclosed in US2006/024433 (Blessing), US2008/0312617 and US2010/0051166A1 (both to Hundorf et al.) may be used. A method of making channels in such an airfelt-free core is disclosed in WO2012/170798A1 (Jackels et al.) wherein the receiving drum (lay-down drum) which supports the core wrap layer on which the SAP particles are deposited comprises longitudinally mating strips corresponding to the shape of the desired channels that mate with raised trips on the SAP printing drum. WO2015/1253088A1 (Armstrong-Ostle et al.) discloses an improvement to the process in which an auxiliary bonding drum is used to apply pressure by means of mating strips that corresponds to the mating strips on the lay-on drum thus providing for overall better channel bond strength between the adhesive and the core wrap layers in the channel. Similar processes for making the channel bonds but in the context of an airfelt (a mixture of cellulose fibers and SAP particles) absorbent material are known, for example as disclosed in WO2020/188047.

The layer of absorbent material may be typically deposited on at least one layer of the absorbent core serving as substrate, such as the bottom core wrap layer or top core wrap layer. In the SAP-printing process as described in US2008/312,622A1 (Hundorf), a continuous layer of SAP is obtained by depositing SAP on each of the core wrap layers in a pattern having absorbent material land areas separated by absorbent material-free junction areas. The absorbent material land areas of the first layer correspond substantially to the absorbent material-free junction areas of the second layer and vice versa, so that a continuous layer of SAP is obtained when the two discontinuous layers are combined.

The absorbent core may comprise one or more glue layers, in particular an auxiliary glue 70 may be disposed between the internal surface of at least one of the top and bottom core wrap layers and at least a portion of the absorbent layer 46 to adhesively immobilize the absorbent material (SAP) within the core wrap.

The auxiliary glue is typically applied by a suitable glue applicator, typically a slot glue applicator comprising a shim having a regular slot interval to provide for a regular application of the auxiliary glue, on one of core wrap top layer or bottom layer as substrate. The auxiliary glue 70 may extend transversally across the whole width of the absorbent layer or a portion thereof, typically the width of the auxiliary glue 70 is at least 50% the width of the absorbent layer so that a significant proportion of the absorbent material may be at least partially immobilized by the auxiliary glue. However the auxiliary glue does not necessarily extend the longitudinal edges 604, 606 of the absorbent layer.

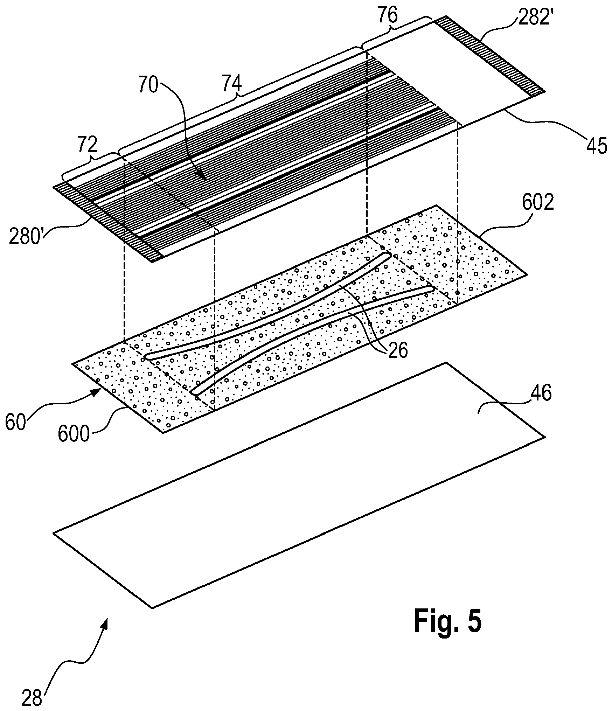

The auxiliary glue 70 typically comprises a series of longitudinal stripes separated by gaps. The auxiliary glue is at least present in the core channel area 74, which is the area of the core longitudinally delimited by the channel. The auxiliary glue may also be present in the core front region 72, which is the area of the core disposed in the front of the channel, and optionally on the core back region 76 which is the area of the core disposed in the back of the channel. As shown in the example of Fig. 5, it may be chosen not to have auxiliary glue in the core back region 76 as this area of the core may comprise relatively less SAP than the rest of the core and thus require less immobilization.

A micro-fibrous thermoplastic adhesive net may also be used in airfelt-free cores as described in the above Hundorf references to immobilize SAP particles on at least one of the substrates formed by the top and bottom core wrap layers. These adhesives are not represented in the Figures for simplicity.

The basis weight (amount deposited per unit of surface) of the superabsorbent material may also be varied to create a profiled distribution of superabsorbent material, in particular in the longitudinal direction to provide more absorbency in the crotch portion of the article, but also in the transversal direction, or both directions of the core.

The core wrap is formed by one or two substrate layers that form the core wrap top and bottom layers that sandwich the SAP particles and a least partially immobilizes the SAP particles so that the absorbent core keeps its integrity. The core wrap top layer 45 and the core wrap bottom layer 46 are also referred to in the art as core cover and dusting layer respectively. These core wrap layers are typically low basis weight nonwoven (typically less than 20 gsm, in particular from 8 gsm to 14 gsm), and may be in particular SMS nonwoven (Spunbond-Meltblown-Spunbond laminate), as is known in the art. The upper and lower core wrap layers may be any material capable of containing and providing a support for the absorbent material.

The core wrap layers may be made of the same or different materials, i.e. two nonwoven webs which have the same of different properties e.g. in a c-wrap configuration. The core wrap may also be made of a single, continuous nonwoven web, which is wrapped around the layer of absorbent material as this may simplify construction and comprising a single longitudinal seal, in this case the top core wrap and the bottom core wrap layers are made of the same web material.

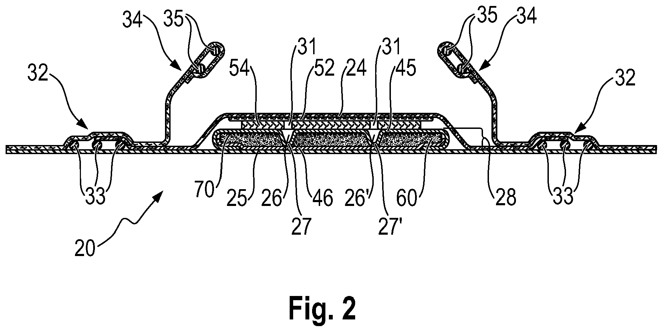

The core wrap layers may be bonded face to face, at least longitudinally as represented in Fig. 2, but other bonding configurations are possible, in particular a C-wrap configuration where one of the top or bottom core wrap layer is larger than the other, so that flaps can be folded around the absorbent material and attached to the other core wrap layer.

The total amount of SAP present in the absorbent core is adapted to the need of the expected wearer of the article. Diapers for newborns require less SAP than infant or adult heavy incontinence diapers. The amount of SAP in the core may be for example comprised from about 2 g to 50 g, in particular from 5 g to 40 g for typical baby diapers. The average SAP basis weight within the absorbent core may be for example of at least 50, 100, 200, 300, 400, 500 g/m² or more, or from 200 g/m² to 400 g/m². The average SAP basis weight is the total amount of SAP in the core divided by the area delimited by the periphery of the SAP layer (including any channels if present).

### Channel(s)

The absorbent core 28 comprises at least one elongated, absorbent material-free channel 26. A channel is an area within the absorbent material layer 60 which is substantially free of absorbent material, so that the core wrap top layer 45 and core wrap bottom layer 46 can be directly bonded to each other through at least a portion of the length of the channel (it is however not excluded that some superabsorbent particles may be deposited accidentally in the channels during core-making). The absorbent core may comprise one or more channels according to the invention, in particular a pair of channels as exemplified, so that the word "channel" as used herein means "one, two or more channel(s)", unless indicated otherwise. The channel preferably does not extend to any of the side of the absorbent layer, and thus is surrounded by the absorbent material.

The absorbent comprises at least one elongated absorbent channel having a length L" which is at least 20% of the length of the absorbent layer L'. Unless otherwise mentioned, all the length reported herein are measured in the longitudinal direction y (i.e. length means the length as measured projected on the longitudinal axis). The length L" of the elongated channel may be of from 20% and 80%, or from 20% to 70%, or from 30% to 60%, of the length L' of the absorbent layer. The elongated core channel may have a length L" of at least 12 cm. The absorbent core may comprise a pair of such channels disposed symmetrically on each side of the longitudinal axis 80, wherein these channels may be straight, curved, or combinations thereof. Such a pair of channels may be disconnected, as illustrated in Fig. 2. The channels may also be connected, for example at one or both their extremities to form a U or O shape. Examples of channel shapes are disclosed in further details in WO2012170778A1, WO2012170781 (Kreuzer et al.). The channel may have other shapes, for example there may be a single straight channel aligned with the longitudinal axis or a channel having a central stem and two or more branches of each of the side of the longitudinal axis.

The article of the invention may comprise at least one elongated, absorbent material-free channel 26 having a front end 62, a back end 66 and a middle point 64. The front end 62 and the back end 66 of the channel are respectively the front extremity and the back extremity of the channel. By "front" and "back", it is meant closer to the front edge of the article and the back edge of the article respectively.

The middle point 64 of the channel is a point of the channel which is disposed longitudinally equidistantly between the front end 62 and the back end 66 of the channel. The middle point 64 is present in the crotch region 37 of the article, so that the channel is generally centered on the crotch region 37 of the article.

The channel comprises a channel bond 27 between the core wrap top layer and the core wrap bottom layer. The bond 27 may extend at least between the middle point 64 and the front end 62 of the channel and provides structural integrity of the channel in the dry state and at least partially in the wet state. When the surrounding absorbent material swells during use, the channel can at least partially form a three-dimensional ditch along the bonded area of the channel. Any known bonding techniques known in the art may be used to provide for this bond, in particular one selected from adhesive bonding, thermo bonding, mechanical bonding, ultrasonic bonding, or any combinations thereof. Adhesive bonding is however preferred as it allows to easily control the channel bond strength without the risk of damaging the core wrap in the channel area.

The channel bond 27 may be at least partially formed by a channel glue deposited on at least one of the inner surface of core wrap top layer or the inner surface of the core wrap bottom layer, and the other core wrap layer is placed in contact with the glue so that a channel glue bond is formed between the bottom and top core wrap layers through the absorbent material-free channel. This channel glue may consist of or comprise of the auxiliary glue 70, as is discussed further below.

According to the invention, it was found beneficial that the channel comprises a front section, a crotch section and an intermediate section having substantially different channel bond strength, as will be detailed and exemplified below. The front section 100 is disposed adjacent or encompassing the front end 62 of the channel. The crotch section 104 of the channel is disposed adjacent or encompassing the center point 64 of the channel, and the intermediate section 102 is disposed between these two sections. The channel bond strength is measured in minutes using the Static Peel Force Time Test, which measures the channel bond survival time of a 2.54 cm (1 inch) wide cut-out core laminate sample (the 2.54 cm are measured in the longitudinal direction). The Static Peel Force Time Test method describes below in more details how the channel sections are sample from the core laminate and how the bond strength is tested.

According to the invention, the bond strength varies along the length of the channel so that the channel bond strength in each of the front section and the crotch section is respectively higher than the bond strength in the intermediate section. The bond strength of each section is measured in minutes according to the Static Peel Force Time Test disclosed hereinbelow.

In other words, the bond strength is lower in the intermediate section than in the front section and the crotch section. The intermediate section has thus by design a weaker bond strength and this section can first release swelling pressure in the core by delamination of the channel bond. The intermediate section is advantageously disposed close to or in the region where the urine is typically first delivered to the diaper (so-called "pee point") so the invention design allows the swelling pressure to be first released in the portion of the core where it is most needed, while keeping the functionality of the channel in the crotch and front region of the diaper.

The channel crotch section 104 encompasses or is adjacent the channel's middle point 64. The crotch section 104 may be in particular longitudinally centered on the middle point 64 of the channel.

The channel front section encompasses or is adjacent the channel's front end 62, in particular the channel front section may be disposed at a longitudinal distance of 23 mm from the front end 62.

The intermediate section 102 is disposed between the front section and the crotch section. According to the invention, at least one sample selected anywhere in the intermediate section should have a channel strength which is lower than the channel strength in the front and crotch sections of the channel. The intermediate section 102 is disposed between the front section 100 and the crotch section 104. At least one intermediate section 102 having a length of 2.54 taken at any point between the front section and the crotch section should have a bond strength as indicated in the claims.

The bond strength is measured in minutes according to the Static Peel Force Time Test described herein.

The intermediate section may have a bond strength of at least 2 minutes, in particular, from 2 minutes to 100 minutes, more particularly from 4 minutes to 50 minutes.

The front section may have a bond strength of at least 20 minutes. The maximum bond strength measurable by test design is 999 minutes, however a maximum bond strength for the front section for practical purpose may be up to 500 min, or up to 400 min, or up to 300 min, or up to 200 min. The front section may thus have a bond strength in the range of from 20 minutes to 500 minutes, or from 20 minutes to 400 minutes, of from 20 minutes to 300 minutes, or from 20 minutes to 200 minutes.

The front section may have a bond strength which is about the same, or preferably higher than the bond strength of the crotch section.

The crotch section may have a bond strength of at least 20 minutes. The crotch section may have a bond strength in the range of from 20 minutes to 500 minutes, or from 20 minutes to 400 minutes, of from 20 minutes to 300 minutes, or from 20 minutes to 200 minutes. The bond strength of the front section may optionally be at least 10 minutes higher than the bond strength of the crotch section, for example from 10 minutes to 200 minutes stronger in the front section than in the crotch section.

The bond strength of the crotch section 104 may be at least twice as strong as the bond strength of the intermediate section 102. The bond strength of the crotch section 104 may in particular be between two times and thirty times as strong as the bond strength of the intermediate section (the bond strength as measured in minutes according to the Static Peel Force Time described herein).

The bond strength of the front section 100 may be at least twice as strong as the bond strength of the intermediate section 102. The bond strength of the front section 100 may in particular be between two times and thirty times as strong as the bond strength of the intermediate section 102 (the bond strength as measured in minutes according to the Static Peel Force Time described herein).

### Adhesive pattern 70

The absorbent core advantageously comprises an adhesive. Typically, hotmelt adhesives are used in core making process. Hotmelt adhesives are solid at room temperature, thus they are typically heated to the molten state when they are applied on a substrate, before the resolidify, which is the so-called open time, a bond can be formed by pressing another substrate with the glue on the first substrate.

The absorbent core on the invention comprises an auxiliary glue 70 that at least partially immobilizes the absorbent material on at least one of the core wrap top layer or bottom layer. This immobilization is relevant before the use of the absorbent material, so that the absorbent material stays in place in the core wrap before being used. The auxiliary glue may be applied on the inner side of either the core wrap top layer and/or core wrap bottom layer, typically by slot glue application or any other means.

The adhesive may also be present in the core wrap at least in the channel area 74 and at least partially forms the channel bond 27 between the core wrap top layer and core wrap bottom layer. By applying pressure in the channel area 74 during the core making process, the two layers of the core wrap are contacted through the channels and the adhesive provides an adhesive bond between these two layers through the channel. Exemplary patent disclosures of such adhesive bonding processes can be found for an airfelt or airfelt-free absorbent cores in WO2012/170798A1 (Jackels et al.), EP2,905,000 (Jackels et al.) and EP2,905,001 (Armstrong-Ostle et al.).

The varying bond strength between the different channel sections may be obtained by varying the amount of glue along the length of the channel. In this case, the front section and crotch section may respectively comprise more glue than the intermediate section, per unit of channel area. The glue pattern may be varied along the channels according to the following examples.

The auxiliary glue 70 may advantageously also be used to form the channel bond 27. When the channel 26 is at least partially curved or angled relative to the longitudinal axis (in other words when the channel is not straight and parallel to the longitudinal direction) at least between the front end 62 and the middle point 64 of the channel, the auxiliary glue 70 may be applied in an irregular pattern to provide for the different channel bond strength in the front, intermediate and crotch section of the channel. This may be a particularly simple and effective way to provide the different strength values in the channel, in particular where the channel is curved (as illustrated in Figure 5) or straight but at an angle relative to the longitudinal axis.

Contrary to conventional auxiliary glue pattern which comprises a regular series of longitudinal stripes having uniform width and distance between stripes (typically 1 mm), the stripes of auxiliary glue 70 according to this irregular pattern may have different widths in the channel area 74, so that due to the curvature or angle of the channel relative to the longitudinal axis, the channel crosses larger or thinner stripes of glue in each of the front, intermediate and central sections. Alternatively or additionally, the auxiliary glue pattern may comprise varying gaps between the stripes. In this way, the area of the channel that intersects the stripes with the smaller gaps will have a higher adhesive content as the area of the channel that intersect the stripes having a larger gaps between them.

An auxiliary glue pattern 70 that may be used to form the channel bond 27 according to the invention is illustrated in Figs. 5-6, the latter showing a close-up view of a longitudinal stripes pattern of the auxiliary glue 70. This auxiliary glue 70 may be applied on the core wrap top layer 45, or the core wrap bottom layer 46, or even both layers. The shape of the channel 26 is shown in dotted lines. The front, intermediate and crotch sections 100, 102, 104 respectively are shown as well in dotted lines.

The auxiliary glue stripes may comprise one or more first stripe(s) 82 having a first width and one or more second stripe(s) 84 having a second width, wherein the second width is larger than the first width. The second width of the second stripe 84 may be at least twice, or at least three times, larger than the first width of the first stripes 82.The transversal gap, or distance, between the stripes may also be varied to adjust the amount of glue in each channel sections considered. The gap between the stripes may thus comprise a first gap 86 having a first gap width, a second gap 88 having a second gap width, wherein the second gap width 88 is larger than the first gap width 86, for example the second gap width may be at least twice, or three times or more, larger than the first gap width 86.

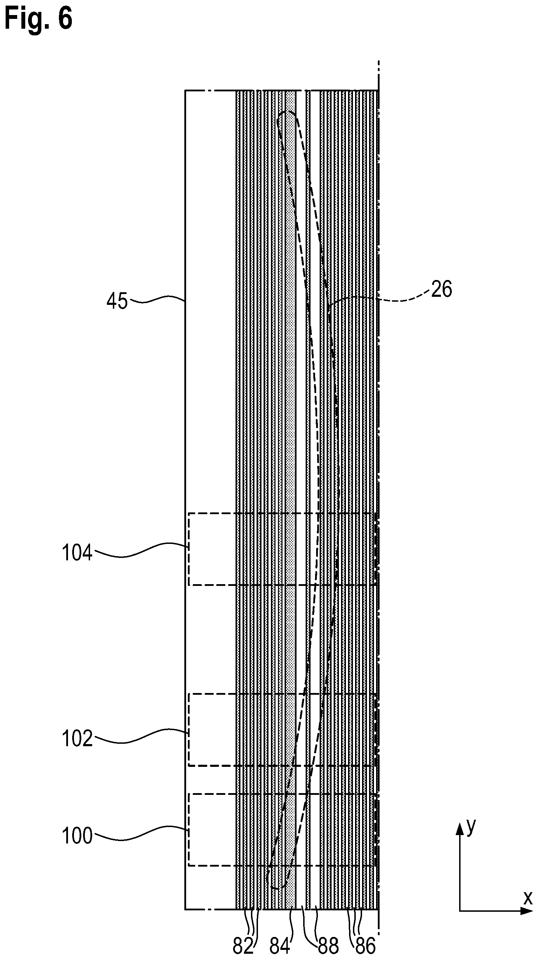

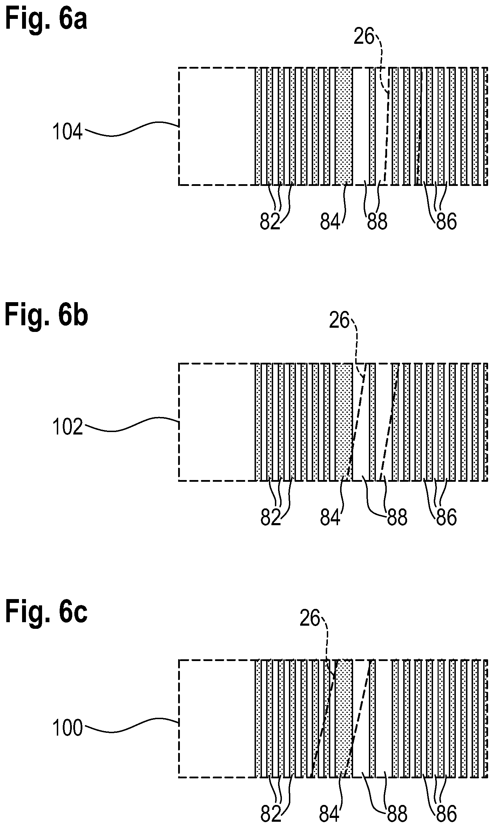

By disposing the larger glue stripe 84 to intersect with the front section of the channel 26, a larger amount of glue per channel area is provided in the front section 100, as illustrated in a close-up view on Fig. 6c. Similarly, by transversally disposing the larger gap area(s) 88 so that they intersect with the intermediate section of the channel, less glue per channel area is present in this section resulting in the weaker channel bond, as shown in Fig. 6b for the intermediate section 102. The longitudinal stripes 86 having the smaller width separated by the smaller gaps 88 may intersect with the crotch section of the channel, as shown for the crotch section 104 in Fig. 6a, resulting in a medium channel bond strength, which is stronger than in the intermediate section but may be weaker than in the front section. It is also to be noted that the bond strength may be disproportionally increased with the stripe width due to a better uniformity of the glue in larger stripes, for example it was noticed that a pair of 1 mm stripe separated by a 1 mm gap can provide a bond weaker than a 2 mm wide stripe.

Using an auxiliary glue 70 pattern which comprises a series of longitudinal glue stripes 82, 84, wherein the stripes have different width and/or gap to vary the channel bond strength along the length of the channel was found to be simple and cost-efficient. Regularly disposed longitudinal glue stripes applied from a shim are a standard glue application pattern typically used for auxiliary glue adhesive. Thus, the channel bond design may be obtained simply by adjusting the shim pattern, from having a regular width and gaps to an irregular successions of width and/or gaps adapted to the channel design considered.

Using such an irregular auxiliary glue pattern of longitudinal stripes may however not be directly applicable to a straight channel which is parallel to the longitudinal direction. In that case, it may be required to use an irregular, transversally oriented auxiliarily glue pattern to achieve the same effect.

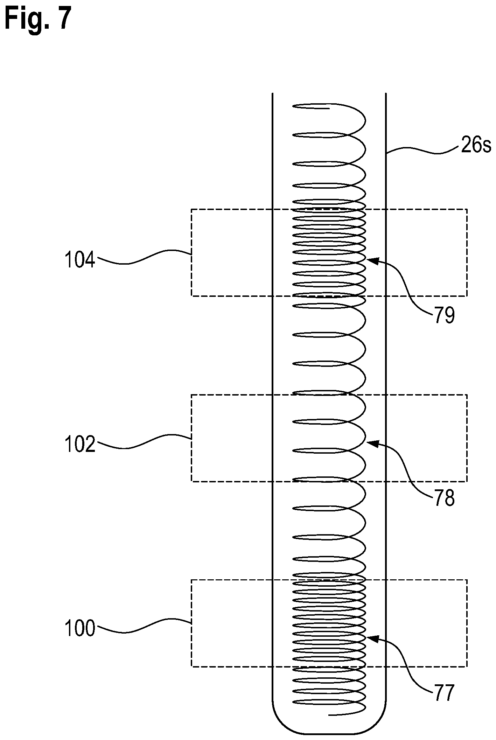

Fig. 7 shows an alternative glue application pattern which can alternatively be put in practice for such a straight channel 26s. In this alternative execution, the channel glue can be applied using an applicator than can vary the amount of glue discharged along the length of the channel. The example of Fig. 7 shows a glue applied in a spiral pattern, wherein the numbers of swirl per unit of length of the spiral is high in the front section 100, medium in the crotch section 104 and low in the intermediate section 102. More generally, the glue pattern has an area 77 of relatively high amount of glue overlapping with the front section 100, an area 79 of medium high amount of glue overlapping the crotch section 104 and an area 78 of relatively low amount of glue in the intermediate section. Glue applicators capable of varying the amount of glue deposited are for example disclosed in US2005/0,137,549A1 (Lindsay et al.) and are a good practical solution for straight channels which are parallel to the longitudinal direction.

While using an irregular auxiliary glue pattern may be a preferred method for obtaining the channel bond of the invention, other possible executions may be used for the same purpose.

Another possible execution involves applying varying pressure along the different sections of the channels so that the channel bond strength varies according to the pressure pattern. In a core channel making process (see references above), pressure is typically applied in the core channel area 74 so that a sufficient contact between the core wrap layers and the glue is attained during the open time of the glue, thus improving the channel bond strength in the channel area.

Generally, in the channel bonding process, the core is briefly pressed between a mating strip on a first roll and another mating strip on a second roll or alternatively a smooth roll, so that the glue can effectively attach both layers of the core wrap through the channels, see for example in WO2012/170798A1 (Jackels et al.) and WO2015/1253088A1 (Armstrong-Ostle et al.) which discloses an auxiliary bonding drum used to apply pressure by means of mating strips that corresponds to the mating strips on the lay-on drum).

The bond strength can also be varied along the channel (independently of the channel shape) by providing a channel bonding tool that applies a varying pressure along the length of the channel. One or both these mating strips may be machined in relation with the channel bonding process disclosed so that the profile of the mating strip comprises a small but effective depression in a section corresponding to the intermediate section of the channel, so that less pressure is applied to the core channel in this section. The profile may be further varied to provide more pressure in the crotch section than in the front section.

So in this execution, it is possible to apply a regular pattern of longitudinal stripes of auxiliary glue in the channel area 74, for example as an auxiliary glue comprising a regular series of 1 mm longitudinally extending stripe of glue separated by equally 1 mm wide gaps, and apply each section of the channel with different pressure in the bonding process so that the bonding pattern of the invention is obtained. The shape of the channel (straight or curved) is not limited in this execution.

### Process

The channel bond 27 may be at least partially formed by the auxiliary glue 70 deposited on at least one of the inner surface of core wrap top layer or the inner surface of the core wrap bottom layer in the channel area 74. The process of making a core and forming the core wrap bond as indicated above may comprise the steps of:
providing a first core wrap substrate;
applying a first auxiliary glue on the first core wrap substrate;
wherein the first auxiliary glue comprises a plurality of parallel auxiliary glue stripes, wherein two adjacent stripes are separated by a gap;
depositing a first absorbent material on the first core wrap substrate,
wherein the first absorbent material is at least partially immobilized by the first auxiliary glue;
wherein the first absorbent material layer formed by the first absorbent material comprises at least one elongated absorbent material-free channel;
providing a second core wrap substrate;
combining the layers above with the second core wrap substrate to form an absorbent core.

Optionally a second auxiliary glue and/or a second absorbent layer may also be applied on the second core wrap substrate. The first and second auxiliary glue may each have an irregular pattern as described herein, or it may be that the combination of the first and the second auxiliary glue for the auxiliary glue pattern of the invention. This optional choice may be more costly because as second glue applicator may be needed but a better absorbent material immobilization may be provided as the auxiliary glue is applied from both sides of the absorbent material layer. In any case, the auxiliary glue pattern obtained is irregular pattern, as will be described in further details below.

Of course other steps may be present in the process of making the absorbent core. For example, for airfelt-free cores made using SAP printing technology, the absorbent material will typically by applied as one half layer on each core wrap substrate and comprise an additional microfibrous net to immobilize each half layer of absorbent material, as described above. An airfelt core (comprising a mix of cellulose fibers and superabsorbent particles) on the other hand may be sufficiently immobilized by one auxiliary glue layer, whereas the combination of a first and second auxiliary glue layers is also possible.

Typically, hotmelt adhesives are used in core making process as auxiliary glues or microfibrous glue. Hotmelt adhesives are solid at room temperature, thus they are typically heated to the molten state when they are applied on a substrate, before the resolidify, which is the so-called open time, a bond can be formed by pressing another substrate with the glue on the first substrate.

### Examples

The present invention is applicable to a wide range of absorbent cores and absorbent articles. The dimensions and materials used may vary depending on the intended applications as is known in the art. For example, absorbent cores for larger size diapers will typically be longer and larger and comprise more absorbent material than absorbent cores for smaller size diapers.

### Example 1:

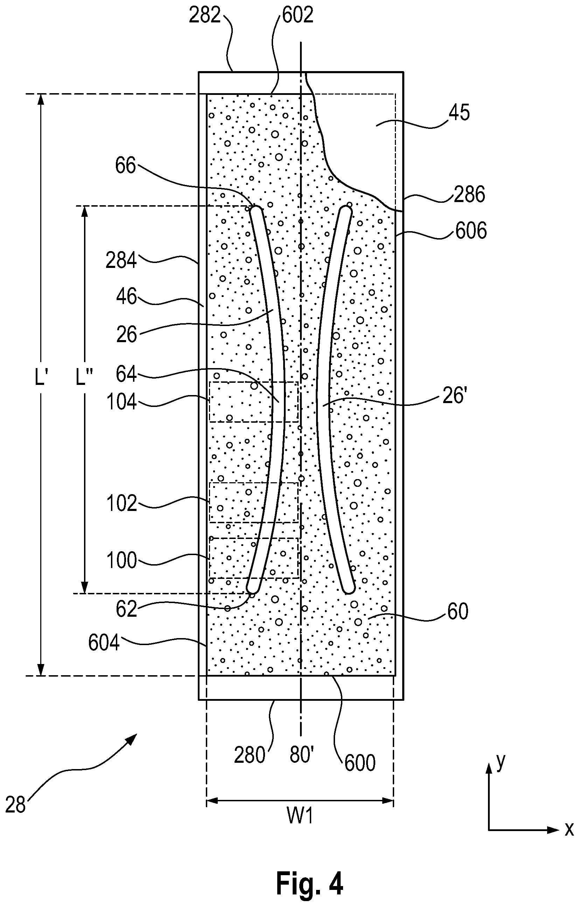

The following illustrates dimensions and materials for an exemplary article and absorbent core as represented in Figs.1-6 and comprising a pair of core channels and a pair of distribution channels in the distribution layer. The dimensions indicated are for a "size 4" ("Midi") diaper, which can be recommended for baby weighing between 7 kg and 18 kg. All widths are measured parallel to the transversal direction (x) and all lengths are measured parallel to the longitudinal direction (y).

| | |
|---|---|
| L (length of the article) | 478 mm |
| L' (length of the absorbent layer) | 360 mm |
| W1 (width of the absorbent layer) | 110 mm |
| Width of the core (including core wrap) | 120 mm |
| L" (length of the channel) | 182 mm |
| Width of the core channels 26 | 8 mm |

The core wrap comprises a top layer C-wrapped around a bottom layer with adhesive longitudinal seals (as in Fig. 2). The top layer has a width of 165 mm, the bottom layer has a width of 130 mm. The final core bag's width is approximately 120 mm. The top layer is an 8 gsm water permeable propylene spunbonded nonwoven (from Fitesa Germany) and the bottom layer a hydrophobic, 10 gsm nonwoven (from Fibertex). The core wrap is sealed by gluing at its front edge and its back edge. The absorbent material consists entirely of SAP particles, which are deposited for one half on the top layer and the other half on the bottom layer of the core wrap and immobilized by a microfibrous glue (SAP printing method as described above). An auxiliary slot glue layer is disposed on the inner side of the top core wrap layer with an overall width of 81 mm and a pattern of alternating stripes as represented in Fig. 6 (small stripes 82 are 1 mm wide, the large stripe 84 is 3 mm wide, the small gap 86 are 1 mm wide and large gaps 86 are 3 mm wide). The total amount of SAP in the core is 12.8 g. The absorbent layer had a generally rectangular shape. The basis weight of the SAP in the absorbent layer is longitudinally profiled so that more absorbency is available towards the middle and the front of the core. The total length of the core wrap is about 10 mm longer in the front and in the back than the length of the absorbent material layer to provide for front and back core wrap end seals 280', 282'.

The diaper incorporating the absorbent core comprises a dual acquisition-distribution system comprising a latex bonded, nonwoven acquisition layer 52 and a cross-linked cellulose fibers distribution layer 54 between the acquisition layer and the absorbent core, having a shape as generally illustrated in Figs. 1-6 as described above. The width of the rectangular acquisition layer 52 is 105 mm and the maximum width of the distribution layer 54 in this example is 80 mm.

The comparative diapers have the specification of a size 4 commercial diapers (Baby-Dry^{®} taped diaper Western Europe) and were manufactured on the same converting line as the example diapers. The comparative diapers had channel in the core and the distribution layer having the same shape as the inventive diapers. The comparative diapers each comprised 13.1 g of SAP compared to the invention example 12.8 g of the same SAP. The comparative diapers had a rectangular absorbent layer having a 110 mm width and a substantially rectangular distribution layer having a width of 80 mm (also including a rounded back edge as in the diaper example). The other main difference was that the comparative example had a regular auxiliary glue pattern, comprising a succession of 1 mm stripes separated by 1 mm gaps over a width of 81 mm instead of the above-described auxiliary glue pattern of the invention. The core construction was otherwise similar for example 1 and comparative example.

### Testing:

The channel bond strength in the different sections of the channels was measured according to the Static Peel Force Time Test described below. The results are indicated in table 1 below.

**Table 1**

| | Static Peel Force Time (min) | | |
|---|---|---|---|
| | Front Section | Intermediate Section | Crotch Section |
| Comparative example: left channel | 21 | 41 | 54 |
| Invention example: left channel | 56 | 17 | 68 |
| Comparative example: right channel | 35 | 82 | 91 |
| Invention example: right channel | 92 | 37 | 51 |

The comparative and inventive diapers were put on a baby sized articulated mannequin having a fluid inlet in the crotch allowing gushes of saline solution to simulate gushes of urine. A first gush of 150 ml urine was injected with the mannequin in a standing position. The mannequin was then tilted forward, simulating a baby laying on the belly. A series of 25 ml gushes with 2-minute pauses between each subsequent gush were then injected until the diaper starts leaking at the front. The measurements were repeated on 5 diapers for each diaper examples.

The inventive example showed a higher average urine load at failure in this front leakage protocol (+15 g load overall diaper and +18 g load measured in the core). This is excellent considering the inventive example comprised 0.44 g less SAP than the comparative example.

The diapers were further tested for run-off values under laboratory conditions. The diapers were attached to a sloped support with their front edge on the high side of the slope. Then 75 g saline solution was applied at a distance of 170 mm from the front edge to the diaper. Following this, 3 x 75 g of saline solution were further applied at the same point with a 5-minute pause between each gush. The amount of liquid not absorbed (run-off) is measured in g after each of the fluid application. Eight samples were tested for each of the diaper examples.

In a second series of run-off measurements, inventive and comparative diapers were tested again but in a reversed position so that the back edge of the diaper is placed high, and the topsheet was removed to focus the measurements on the intermediate and final storage capacity of the core and acquisition-distribution layer. The liquid was again applied at 170 mm from the front core edge and 4 x 75 g of saline solution were applied and 8 samples were measured for each examples. The averaged results were reported in Table 2 below.

**Table 2**

| | Comparative Example | Inventive example |
|---|---|---|
| Topsheet run-off (Regular method) | 2 / 1 / 12 / 39 (54) | 2 / 1 / 9 / 26s (38s) |
| Gush 1 / 2 / 3 / 4 (total) in g | | |
| Reversed run-off (without Topsheet) | 0 / 1 / 11 / 24 / 35 (236) | 0 / 0 / 9 / 21 / 31 (243s) |
| Gush 1 / 2 / 3 / 4 (total) in g | | |

The suffix "s" in Table 2 indicates statistically significant differences at 95% confidence between the inventive and the comparative examples. The inventive example had statistically significant lower run-off for the fourth and last gushes in the regular run-off test, and for the last gush in the reversed run-off test. Also the core load was higher in the reversed run-off test for the inventive example, despite the absorbent core comprising 0.3 g less SAP, indicating a more effective utilization of the absorbent material.

### Test methods

The values indicated herein are measured according to the methods indicated herein below, unless specified otherwise. All measurements are performed at 22°C ± 2°C and 50% ± 5% RH, and samples should be kept at least 24 hours outside of the bag in these conditions to equilibrate before conducting the tests, unless indicated otherwise.

### Static Peel Force Time Test

The purpose of the method is to assess the survivability of a channel bond sample. This test method measures in minutes how long the channel bond is able to withstand a constantly applied vertical peel force of about 150 grams under standardized conditions (Static Peel Force Time).

### Equipment

| | |
|---|---|
| Medium Clip | Medium Binder Clips 25 mm Capacity (#72050). ACCO World Product. Other suppliers: Yihai Products (#Y10003), Universal Office Products (#10210), Diamond (#977114), or equivalent. The clip weights 4.5 g +/-1 g. |
| Large Clip | Large Binder Clips 2 inch (50.8mm). ACCO World Product. Other suppliers: Yihai Products, Universal Office Products, Diamond, or equivalent |
| Test Stand | RT-10 room temperature (Shear Tester) w/ timer. Chem Instruments, 510 Commercial Drive, Fairfield Ohio 45014-9797, USA; or equivalent. Must be placed in a vibration free area. |
| Weight | 150g (+/- 1g) TW150 Shear Tester Weight with hook on top (to attach to the clip). Chem Instruments, 510 Commercial Drive, Fairfield Ohio 45014-9797, USA; or equivalent |
| Cutting Tools | Scissors and a 25.4 mm (1 inch) cutter (convenient source, e.g. JDC Precision Sample Cutter made by Thwings-Albert Instrument Company Philadelphia USA, cat# 99, cut width 25.4 mm, accuracy at least +/- 0.1 mm) |
| Metal Ruler | Traceable to NIST, DIN, JIS or other comparable National Standard, graduated in mm, longer than the length to be measured. |
| Temperature | Testo-temperature device (or equivalent) to measure temperature at sample height with an accuracy of ± 0.5 °C and ± 2.5 % RH in the range between -10°C and +50°C. Testo GmbH & Co., Postbox 1140, D-79849 Lenzkirch (www.testo.com) Article number for Testo 625: 0563 6251. |

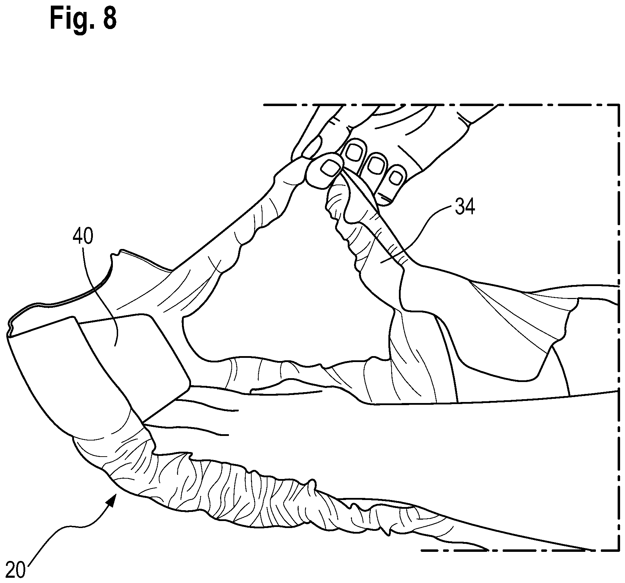

### Core preparation (see Fig. 8):

The absorbent articles are left at least one week in their closed package after production in order for the bond strength to stabilize. The absorbent core can be extracted from a commercial article (in the following a diaper, but the method applicable to other type of absorbent articles) as follows:
1. Open the diaper topsheet side up and place the diaper flat onto a table. For pants, open the side seams and remove the waistbands. Hold the diaper with one hand and carefully remove the ears (40) and the barrier cuffs (34) along the cuffs continuous bond (outer edge) on both sides of the articles.
2. Gently remove topsheet and acquisition system without damaging the core end seals, if present. The backsheet does not need to be removed.

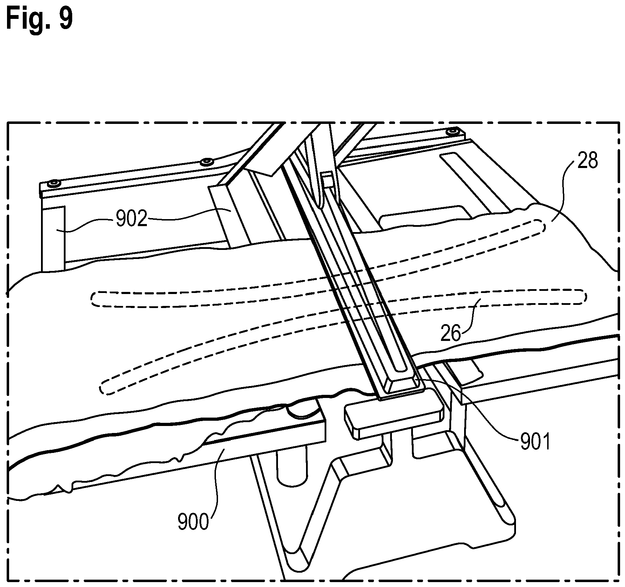

### Channel Sample preparation (see Fig. 9):

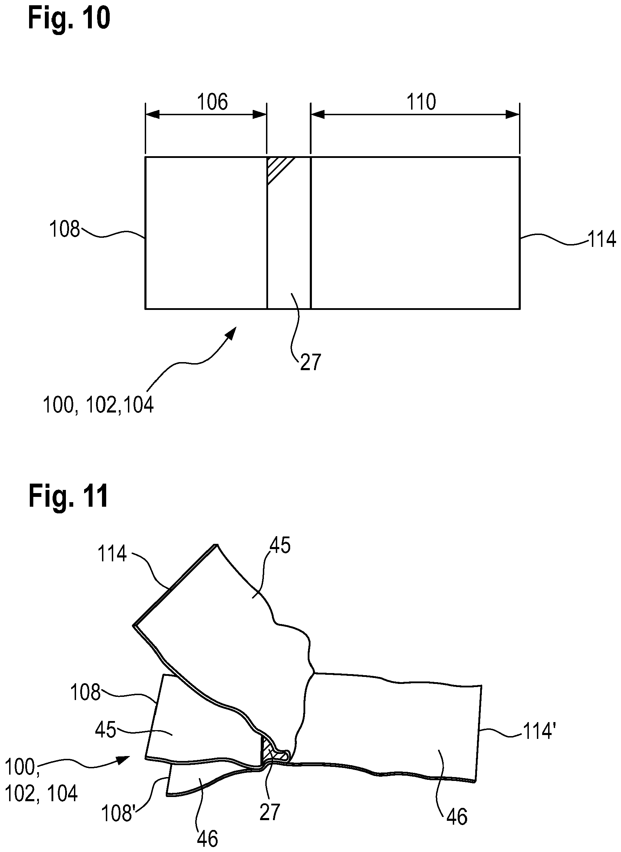

For each channel proceeds as follows:
1. Put the core under a lamp table or a UV-light to identify the beginning (62) and the end (66) of the channel.
2. Define the longitudinal middle point (64) of the core channel (26) by using a ruler.
3. Mark a crotch transversal line perpendicular to the longitudinal axis passing through the middle point (64) of the channel. Similarly mark a front transversal line at the desired position towards the front end of the channel, in particular the front transversal line may be disposed at distance of 23 mm in the longitudinal direction from the front end (62) of the channel, and an intermediate transversal line at a desired position in the intermediate section between the front transversal line and the crotch transversal. In particular, the intermediate transversal line may be disposed at a distance of 23 mm from the front transversal line. These transversal lines are used to center the cutter blade that will cut a 25.4 mm (1 inch) wide band of the core laminate in each of the front, middle and intermediate section.
4. Lay the core (28) on a supporting table (900) fitted with the 25.4 mm wide cutter (901) and align to the crotch transversal line of the channel. Double sided tapes (902) may be used to keep the sample in place. The table comprises two grooves or gaps on each side of the area to be cut so that the cutter blade can penetrate through the thickness of the core.
5. Cut the core in transversal direction to obtain a band of core material comprising at least one channel, optionally check the width of the cut sample band (target = 25.4 ± 2 mm).
6. Repeat the two preceding steps 4 and 5 for the front transversal line and the intermediate transversal line, so that in total three bands are obtained representative of the front, intermediate and middle section of the channel.
7. The band of core laminate may comprise two (or more) channel bonds per band. In this case, use the scissors to cut the 25.4 mm wide (1-inch) band in the longitudinal direction to obtain at least one channel sample (100, 102, 104) comprising the channel bond (27) representative of each sections. If there are more than one channel per band, note for each channel sample which channel they correspond to (e.g. left or right channel). There should be at least a 5 mm channel-free flap (106) between the channel bond (27) and the inner edge (108) of the sample to ensure proper manipulation of the sample- see Fig. 10. Obtain a left channel bond sample and right channel bond sample if applicable. Label the cut channel bond samples appropriately, e.g. left/right channels.
8. The sample will be clamped on their outer edge (114) during the test. The outer edge (114) of the sample can be trimmed with the scissors, however in order to carry the test, the minimum distance (110) between the channel bond (27) and the outer edge (103) for the sample should be sufficient to ensure a proper clamping of the core wrap material into the clamps. Typically a minimum distance (110) between the channel and the outer edge should be of at least 5 mm for this purpose, 20 mm being ideal.
9. Gently remove any absorbent material outside the channel bond that is between the core wrap top layer (45) and the core wrap bottom layer (46). For this, it may be necessary to open any core wrap longitudinal side seals on the outer edge (114) of the sample, if needed (see Fig. 11).

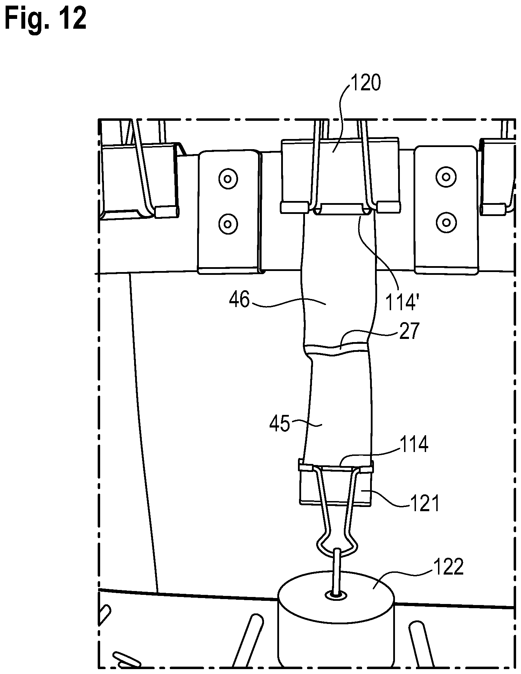

### Test Procedure (see Fig. 12):

Set up the tester in an area where the temperature is constant at 23°C and ensure that the tester have at least 2h time to reach this temperature.
1. Clamp the outer edge (114') of the bottom layer (46) of the sample into the jaw of the large binder (120) clip hanging at the top of the tester bar.
2. Clamp the other binder clip (medium size) (121) to the top layer (45) of the core wrap at its outer edge (114). The inner edge (108) of the sample is facing away from the experimenter.
3. Gently attach the 150 g weight (122) to the medium binder clip and lower the clamped weight slowly until the weight hangs freely on the test sample. Avoid any sudden drop of the weight that could.
4. As soon as the weight is released, push the timer reset button for that sample to begin the timer at 0 minutes. NOTE: The timer must be checked to ensure that it has begun counting from 0.0 min. The operator should look for the number to change from 0.0 min to 0.1min.
5. Repeat procedure above for each sample prepared. The Test Stand allows testing several samples in parallel.
6. The timers will stop once the sample weight has fallen on the bottom plate due to the bond breaking. The bottom plate comprises a switch linked to the timer so that it automatically stops when the weight has fallen down. The time recorded is the Static Peel Force Time for that sample (expressed in minutes). Note: if the weight falls down due to the sample slipping out of the clip (121) or due to core wrap tear without the bond breaking, the test needs to be rerun on a new sample.
7. If the sample weight has not fallen after 999 minutes, the Static Peel Force Time is reported to be 999 minutes (maximum Static Peel Force Time).
8. For a commercial article of a given construction, the measurements are repeated 10 times on equivalent articles (e.g. 10 randomly selected diapers in a commercially-sourced diaper package ) to obtain an average value of the Static Peel Force Time for a given channel section. If the absorbent core comprises two or more channels, the measurements may be conducted for each of the channel separately. For example, if the core comprises a right channel and a left channel, the Static Peel Force Time are measured and reported for the right and left channels separately.

### Misc

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "preferably", "advantageously", "in particular" and the likes also qualify features which are not intended to limit the scope of the claims unless specifically indicated to do so.

Unless indicated otherwise, the description and claims refer to the absorbent core and article before use (i.e. dry, and not loaded with a fluid) and conditioned at least 24 hours at 23 °C +/- 2 °C and 50 +/- 5% Relative Humidity (RH).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A personal hygiene absorbent article (20) comprising a front waist region (36), a crotch region (37), a back waist region (38), a longitudinal centerline (80) extending in a longitudinal direction (y) and a length L measured along the longitudinal centerline, wherein the front waist region, crotch region and back waist region have the same length of one third of L as measured along the longitudinal centerline;
the article comprising a topsheet (24), a backsheet (25) and an absorbent core (28) between the topsheet and the backsheet;
the absorbent core comprising:
a core wrap top layer (45);
a core wrap bottom layer (46);
an absorbent material layer (60) between the core wrap top layer and bottom layer, wherein the absorbent layer (60) has a length L' measured in the longitudinal direction along the longitudinal centerline (80); and
at least one elongated, absorbent material-free channel (26) having a front end (62) and a back end (66) and a middle point (64), wherein the middle point is disposed longitudinally equidistantly between the front end and the back end of the channel, wherein the middle point (64) is present in the crotch region (37) of the article, and the channel has a length L" measuring in the longitudinal direction (y) of at least 20% of L' and/or at least 12 cm;
wherein the channel comprises a channel bond (27) between the core wrap top layer (45) and the core wrap bottom layer (46);
wherein the channel comprises a front section (100) encompassing or adjacent the channel's front end, a crotch section (100) encompassing or adjacent the channel's middle point (64), and an intermediate section (102) disposed between the front section and the crotch section;
wherein the bond strength in the front section (100) is higher than the bond strength in the intermediate section (102); and the bond strength in the crotch section (104) is higher than the bond strength in the intermediate section (102); wherein the bond strength is measured in minutes for each section according to the Static Peel Force Time Test disclosed herein.

2. An absorbent article (20) according to claim 1, wherein the bond strength in the front section (100) is higher than the bond strength in crotch section (104).

3. An article according to any of the preceding claims, wherein the channel length L" is in the range of from 20% to 80% of the length L' of the absorbent layer.

4. An article according to any of the preceding claims, wherein the intermediate section (102) has a bond strength of at least 2 minutes, in particular wherein the intermediate section has a bond strength in the range of from 2 minutes to 20 minutes.

5. An article according to any of the preceding claims, wherein the bond strength of the front section is at least 20 minutes, in particular at least 30 minutes.

6. An article according to any of the preceding claims, wherein the bond strength of the crotch section is at least 20 minutes, in particular at least 30 minutes.

7. An article according to any of the preceding claims, wherein the front section has a bond strength which is at least 10 minutes higher than the bond strength of the crotch section.

8. An article according to any of the preceding claims, wherein the crotch section has a bond strength which is at least 10 minutes higher than the bond strength of intermediate section.

9. An article according to any of the preceding claims, where the length L' of the absorbent layer is in the range of from 50% to 100% of the length L of the article, in particular wherein L' is in the range of from 60% to 90% of L.

10. An article according to any of the preceding claims, wherein the channel bond (27) is at least partially formed by a channel glue (70) between the core wrap top layer and the core wrap bottom layer, wherein the amount of glue per area of channel varies in the different sections, so that the front section and crotch section of the channel each comprises more channel glue than the intermediate section.

11. An article according to the preceding claim, wherein the front section comprises more channel glue than the crotch section.

12. An article according to the preceding claim, wherein the channel is not straight and parallel to the longitudinal direction at least between the front end (62) and the middle point (64) of the channel, and the channel glue is an auxiliary glue (70) which comprises a plurality of longitudinally-oriented parallel stripes (82, 84) separated by gaps (86, 88), wherein the stripes have different width and/or different gap widths, so that a different amount of auxiliary glue per area of channels is present in the front, intermediate and crotch sections of the channel.

13. An article according to the preceding claim, wherein the auxiliary glue (70) is applied on a channel area (74) which is wider in the transversal direction than the channel(s) (26) and the auxiliary glue (70) at least partially immobilizes the absorbent material within the core wrap, preferably wherein the auxiliary glue is also applied on an channel front region (72) of the core wrap disposed in the front of the channel(s), and optionally on an core back region (76) disposed in the back of the channel(s).

14. An article according to claim 10, wherein the channel glue is applied as a spiral glue (77, 78, 79) at a higher basis weight in the front section (100) and crotch section (102) than in the intermediate section (104), and optionally wherein the basis weight of the spiral glue is higher in the front section (100) than in the crotch section (104), in particular wherein the channel (26s) is straight and parallel to the longitudinal direction at least between the front end (62) and the middle point (64) of the channel (26).

15. An article according to claim 10, wherein the channel bond is an hotmelt glue bond formed by applying different pressure between the top core wrap layer and the bottom core wrap layer during the open time of the hotmelt glue so that the varying bond strength in the front, crotch and intermediate sections of the channel are obtained.
